Europäisches Patentamt

European Patent Office

Office européen des brevets

(11) Veröffentlichungsnummer: **0 490 041 A1**

# EUROPÄISCHE PATENTANMELDUNG

(21) Anmeldenummer: **91117181.7**

(22) Anmeldetag: **09.10.91**

(51) Int. Cl.5: **A61K 7/50**, A61K 7/08

(30) Priorität: **08.12.90 DE 4039229**

(43) Veröffentlichungstag der Anmeldung:
**17.06.92 Patentblatt 92/25**

(84) Benannte Vertragsstaaten:
**AT BE CH DE DK ES FR GB IT LI NL SE**

(71) Anmelder: **HÜLS AKTIENGESELLSCHAFT
Patentabteilung / PB 15 - Postfach 13 20
W-4370 Marl 1(DE)**

(72) Erfinder: **Der Erfinder hat auf seine Nennung
verzichtet**

(54) **Kosmetische Reinigungsflüssigkeit.**

(57) Moderne Shampoos oder Badegele bestehen heute im tensidischen Anteil hauptsächlich aus Tensiden auf petrochemischer Basis; die biologische Abbaubarkeit sowie die Ökotoxizität entspricht meist nicht den Tensiden auf nativer Rohstoffbasis. Es wird daher eine kosmetische Reinigungsflüssigkeit vorgeschlagen, die einen sehr hohen Anteil an Tensiden aus nachwachsenden Rohstoffen enthält; die Eigenschaften bezüglich der Verdickbarkeit sowie der Haut- und Schleimhautverträglichkeit sind hervorragend; diese Mischungen besitzen weiterhin eine hervorragende Reinigungskraft, ideales Schaumvermögen und günstige Lösungseigenschaften.

Die erfindungsgemäßen kosmetischen Reinigungsflüssigkeiten bestehen aus einem hohen Anteil an Alkylpolyglycosiden sowie geringen Mengen Sulfattensiden und üblichen weiteren Tensiden.

EP 0 490 041 A1

Die Erfindung betrifft eine umweltfreundliche kosmetische Reinigungsflüssigkeit.

Moderne Produkte wie z. B. Shampoos oder Badegele bestehen heute hauptsächlich aus Fettalkoholethersulfonaten, Fettalkoholsulfaten, Alkansulfonaten, carboxymethylierten Fettalkoholoxethylaten und anderen anionischen oder amphoteren Tensiden.

An diese Shampoos oder Badegele werden hohe Anforderungen bezüglich Schäumverhalten, Verdickbarkeit und insbesondere an die Haut- bzw. Schleimhautverträglichkeit gestellt.

Aufgabe der Erfindung war es daher, eine kosmetische Reinigungsflüssigkeit zur Verfügung Zu stellen, die neben guter Verdickbarkeit und Schäumvermögen auch eine hervorragende Haut- und Schleimhautverträglichkeit aufweist.

Die Aufgabe wurde gelöst durch eine kosmetische Reinigungsflüssigkeit, die neben Sulfattensiden Alkylpolyglycoside enthält.

Gegenstand der Erfindung ist eine kosmetische Reinigungsflüssigkeit, deren tensidischer Anteil 45 bis 90 Gew.-% Alkylpolyglycosid der Formel I

$R-O-Z_n$    I,

wobei R ein gesättigter oder ungesättigter, verzweigter oder unverzweigter Alkylrest mit 10 bis 18 Kohlenstoffatomen, $Z_n$ ein Polyglycosylradikal mit n = 1.5 bis 3 Hexose- oder Pentoseeinheiten oder Mischungen davon bedeuten,

10 bis 55 Gew.-% Sulfattenside und
0 bis 10 Gew.-% übliche weitere Tenside
enthält.

Die Verwendung von Alkylpolyglycosiden in Wasch- und Reinigungsmitteln ist in Kombination mit anderen Tensiden bekannt. So beschreiben Z. B. die EP-0 075 994, EP-0 075 995 und EP-0 075 996 eine Mischung aus APG, die neben anionischen auch nichtionische Tenside enthalten; hierbei liegt der Glycosidierungsgrad des Alkylpolyglycosides bei G ≧ 1,5.

Auch die EP-0 341 071 beschreibt eine APG-haltige Mischung mit anionischen Tensiden und Betainen; hierbei ist der Glycosidierungsgrad des Alkylpolyglycosides G = 1 bis 3. Auch die EP-0 370 312, sowie EP-0 199 765 beschreiben APG-haltige Mischungen in Kombination mit anionischen Tensiden.

Es wurde nun überraschend beobachtet, daß die erfindungsgemäße kosmetische Reinigungsflüssigkeit hervorragende Haut- bzw. Schleimhautverträglichkeiten aufweist, wenn der Alkylpolyglycosidanteil sehr hoch liegt.

Weiterhin konnte überraschend festgestellt werden, daß die erfindungsgemäßen Kombinationen aus APG, Sulfattensiden und gegebenenfalls weiteren Zusätzen eine hervorragende Reinigungskraft, ideales Schäumvermögen und günstige Lösungseigenschaften, insbesondere hohe Viskositäten bei üblichen Tensidkonzentrationen und niedrigem Elektrolytgehalt besitzen.

Alkylpolyglycoside

Erfindungsgemäß eingesetzte Alkylpolyglycoside genügen der Formel I

$R-O-Z_n$    I,

in der R für einen linearen oder verzweigten, gesättigten oder ungesättigten aliphatischen Alkylrest mit 10 bis 18 Kohlenstoffatomen oder Gemische davon und $Z_n$ für einen Polyglycosylrest mit n = 1,5 bis 3 Hexose- oder Pentoseeinheiten oder Gemische davon stehen.

Bevorzugt werden Alkylpolyglycoside mit Fettalkylresten mit 12 bis 16 Kohlenstoffatomen sowie einem Polyglycosylrest von n = 1,5 bis 2,5. Besonders bevorzugt sind Alkylpolyglucoside.

Die erfindungsgemäß eingesetzten Alkylpolyglycoside können nach bekannten Verfahren auf Basis nachwachsender Rohstoffe hergestellt werden. Beispielsweise wird Dextrose in Gegenwart eines sauren Katalysators mit n-Butanol zu Butylpolyglycosidgemischen umgesetzt, welche mit langkettigen Alkoholen ebenfalls in Gegenwart eines sauren Katalysators zu den gewünschten Alklypolyglycosidgemischen umglycosidiert werden.

Die Struktur der Produkte ist in bestimmten Grenzen variierbar. Der Alkylrest R wird durch die Auswahl des langkettigen Alkohols festgelegt. Günstig aus wirtschaftlichen Gründen sind die großtechnisch zugänglichen Tensidalkohole mit 10 bis 18 C-Atomen, insbesondere native Fettalkohole aus der Hydrierung von Fettsäuren bzw. Fettsäurederivaten. Verwendbar sind auch Ziegleralkohole oder Oxoalkohole.

Der Polyglycosylrest $Z_n$ wird einerseits durch die Auswahl des Kohlenhydrats und andererseits durch die Einstellung des mittleren Polymerisationsgrades n z. B. nach DE-OS 19 43 689 festgelegt. Im Prinzip können bekanntlich Polysaccharide, z. B. Stärke, Maltodextrine, Dextrose, Galaktose, Mannose, Xylose etc. eingesetzt werden. Bevorzugt sind die großtechnisch verfügbaren Kohlenhydrate Stärke, Maltodextrine und besonders Dextrose. Da die wirtschaftlich interessanten Alkylpolyglycosidsynthesen nicht regio- und stereoselektiv verlaufen, sind die Alkylpolyglycoside stets Gemische von Oligomeren, die ihrerseits Gemische verschiedener isomerer Formen darstellen. Sie liegen nebeneinander mit $\alpha$- und $\beta$-glycosidischen Bindungen in Pyranose- und Furanoseform vor. Auch die Verknüpfungsstellen zwischen zwei Saccharidresten sind unterschiedlich.

Erfindungsgemäß eingesetzte Alkylpolyglycoside lassen sich auch durch Abmischen von Alkypolyglycosiden mit Alkylmonoglycosiden herstellen. Letztere kann man z. B nach EP-A-0 092 355 mittels polarer Lösemittel, wie Aceton, aus Alkylpolyglycosiden gewinnen bzw. anreichern.

Der Glycosidierungsgrad wird zweckmäßigerweise mittels $^1$H-NMR bestimmt.

Im Vergleich zu allen anderen in Reinigungsmitteln eingesetzten Tensiden gelten die Alkylpolyglycoside als überaus umweltverträglich. So liegt der mittels Kläranlagen-Simulationsmodell/DOC-Analyse bestimmte biologische Abbaugrad für die erfindungsgemäßen Alkylpolyglycoside bei 96 ± 3 %. Diese Zahl ist vor dem Hintergrund zu sehen, daß bei diesem Testverfahren (Totalabbau) bereits ein Abbaugrad > 70 % die Substanz als gut abbaubar indiziert.

Auch die akute orale Toxizität LD 50 (Ratte) sowie die aquatische Toxizität LC 50 (Goldorfe) und EC 50 (Daphnien) und Werten von > 10 000 mg/kg, 12 bzw. 30 mg/l liegen um den Faktor 3 bis 5 günstiger als die entsprechenden Werte der heute wichtigsten Tenside. Ähnliches gilt für die bei Spülmitten besonders wichtige Haut- und Schleimhautverträglichkeit.

Die erfindungsgemäßen Alkylpolyglycoside fallen synthesebedingt als etwa 50%ige wäßrige Lösung an. Die entsprechenden Reinigungsmittel enthalten 6 bis 30 Gew.-% Alkylpolyglycosid in wäßriger Lösung.

## Sulfattenside

Erfindungsgemäß eingesetzte Sulfattenside sind Fettalkoholethersulfate mit einer Alkylkettenlänge von $C_{10}$ bis $C_{20}$ sowie 1 bis 5 mol EO/mol; als Kationen werden Na, K, $NH_4$ und Alkanolammonium eingesetzt. Weitere erfindungsgemäß verwendete Sulfattenside sind Fettalkoholsulfate bei denen die Alkylkettenlänge $C_{10}$ bis $C_{20}$ beträgt und ebenfalls als Kationen Na, K, $NH_4$ oder Alkanolammonium eingesetzt werden. Die erfindungsgemäßen Reinigungsmittel enthalten 4 bis 25 Gew.-% Sulfattenside, vorzugsweise 3 bis 20 % Aniontensid in wäßriger Lösung.

## Weitere Tenside

Erfindungsgemäß eingesetzte weitere Tenside in geringen Mengen sind Fettalkoholoxethylate mit einer Alkylkettenlänge von $C_9$ bis $C_{20}$, bei denen EO-Grade von 4 bis 20 auftreten; weiterhin werden Sulfosuccinate von $C_{10}$- bis $C_{18}$ Fettalkoholoxethylaten mit 2 bis 6 mol EO/mol eingesetzt. In Konzentrationen bis 1,6 Gew.-% können auch $C_{10}$- bis $C_{18}$-Fettsäureethanol- bzw. -propanolamid verwendet werden; ebenso finden $C_{10}$-bis $C_{18}$-Fettalkoholoxethylatphosphate mit 4 bis 12 mol EO/mol sowie deren Gemische Anwendung.

## Nichttensidische Bestandteile

In den erfindungsgemäßen kosmetischen Reinigungsflüssigkeiten finden elektrolytische Verdickungsmittel wie Natriumchlorid, Ammoniumchlorid, Calciumchlorid etc. sowie wasserlösliche verdickende Polymere wie Polyethylenoxid, Polyethylenglycoldifettsäureester etc. Anwendung. Weiter können als nichttenside Bestandteile Eiweißhydrolysate, Chelatbildner, Konservierungsmittel, Duftstoffe, Farbstoffe, Neutralöle sowie weitere für kosmetische Reinigungsflüssigkeiten übliche und typische Zusätze Verwendung finden.

Die Einsatzkonzentrationen betragen für den tensidischen Anteil von 9 bis 50 Gew.-%, vorzugsweise 10 bis 40 Gew.-%.

## Beispiele

Durch die nachfolgenden Beispiele wird die Erfindung erläutert. Zur Charakterisierung der Formulierung wurde der Klarpunkt und die Viskosität bestimmt.

Zur Klarpunktbestimmung wurden 10 g Reinigungsflüssigkeit in einem Shukoffkolben auf -20 °C abgekühlt und sodann erwärmt. Gemessen wird die Temperatur bei völliger Klärung.

Die Viskosität der Flüssigkeit wurde in einem Rotationsviskosimeter (Haake RV 20) bei 25 °C unter Scherraten von ca. 10 sec$^{-1}$ gemessen.

Das Schäumvermögen wurde nach DIN 53 902/1 bei 40 °C bestimmt, wobei auch die Stabilität (Quotient aus Schaumvolumen nach 30 und nach 300 sec.) abgeschätzt wurde.

Die Tabelle zeigt den Vergleich der Eigenschaften der erfindungsgemäßen Formulierungen mit denen anderer bekannter Kombinationen. Deutliche Verbesserungen - insbesondere bei der Viskosität und dem Schäumvermögen - zeigen die Ergebnisse der erfindungsgemäßen Formulierungen im Vergleich mit dem Stand der Technik. So zeigen die Beispiele 4, 5 und 6 bzw. 8 gegenüber 1 (V) und 2 (V) bzw. 7 (V) die überraschende Überlegenheit der Formulierungen mit hohem APG-Gehalt. Und Beispiel 4 im Vergleich zu Beispiel 3 (V) dokumentiert, daß hydrophilere APG-Einstellungen denen etwas hydrophoberen im Schäumvermögen deutlich überlegen sind.

Folgende Abkürzungen wurden in der Tabelle verwendet:

$C_{12}C_{14}$ [G 1.4]     - $C_{12/14}$-Alkylpolyglycosid mit einem Glycosidierungsgrad von 1.4
$C_{12}C_{14}$ [G 1.6]     - $C_{12/14}$-Alkylpolyglycosid mit einem Glycosidierungsgrad von 1.6
MARLINAT$^{(R)}$ 242     - $C_{12}C_{14}$-Fettalkoholethersulfat, Na-Salz mit 2 mol EO/mol
MARLINAT$^{(R)}$ DFK     - $C_{12}C_{14}$-Alkylsulfat
NaCl     - Natriumchlorid

Tabelle:

| Zusammensetzung | Beispiele | | | | | | | | |
|---|---|---|---|---|---|---|---|---|---|
| | 1 (V) | 2 (V) | 3 (V) | 4 | 5 | 6 | 7 (V) | 8 | 9 |
| $C_{12}C_{14}G_{1.4}$ | - | - | 10 | - | - | - | - | - | - |
| $C_{12}C_{14}G_{1.6}$ | 8 | 8 | - | 10 | 10 | 10 | 3 | 9 | 12 |
| MARLINAT(R) 242 | 12 | - | 10 | 10 | 4 | - | 12 | 6 | 8 |
| MARLINAT(R) DFK | - | 12 | - | - | 6 | 10 | - | - | - |
| NaCl | 1 | 1 | 0,8 | 0,8 | 0,8 | 1 | 2 | 2 | 0,3 |
| Rest zu 100 % VE-Wasser | | | | | | | | | |
| Eigenschaften | | | | | | | | | |
| Klarpunkt (°C) | 10 | 11 | 11 | 9 | 9 | 10 | 9 | 9 | 9 |
| Viskosität (mPa·s) | 2000 | 4700 | 3700 | 4200 | 13000 | 15000 | 500 | 8500 | 3600 |
| Schäumvermögen | | | | | | | | | |
| (ml) nach  30 sec. | 550 | 480 | 400 | 550 | 520 | 500 | 550 | 570 | 560 |
| nach 300 sec. | 460 | 450 | 360 | 510 | 500 | 480 | 470 | 500 | 500 |

**Patentansprüche**

1. Kosmetische Reinigungsflüssigkeit deren tensidischer Anteil 45 bis 90 Gew.-% Alkylpolyglycosid der Formel I

$$R\text{-}O\text{-}Z_n \quad I,$$

wobei R ein gesättigter oder ungesättigter, verzweigter oder unverzweigter Alkylrest mit 10 bis 18

Kohlenstoffatomen, $Z_n$ ein Polyglycosylradikal mit n = 1.5 bis 3 Hexose- oder Pentoseeinheiten oder Mischungen davon bedeuten,

10 bis 55 Gew.-% Sulfattensid und

0 bis 10 Gew.-% weitere Tenside

enthält.

2. Kosmetische Reinigungsflüssigkeit nach Anspruch 1 oder 2,
dadurch gekennzeichnet,
daß das Sulfattensid ein oder mehrere $C_{10}$ - $C_{25}$-Fettalkoholethersulfate mit 1 bis 5 mol Ethylenoxid/mol allein oder in Abmischung mit $C_{10}C_{20}$-Fettalkoholsulfat bedeuten, wobei Na, K, $NH_4$, Alkylammonium als Kationen Verwendung finden.

3. Kosmetische Reinigungsflüssigkeit nach den Ansprüchen 1 bis 3,
dadurch gekennzeichnet,
daß als weitere Tenside $C_9C_{20}$-Fettalkoholoxethylate mit 4 bis 20 mol EO/mol, Sulfosuccinate von $C_{10}$- bis $C_{18}$-Fettalkoholoxethylate mit 2 bis 6 mol EO/mol und/oder bis zu 1.6 Gew.-% $C_{10}$- bis $C_{18}$-Fettsäureethanol- bzw. propanolamide eingesetzt werden.

4. Kosmetische Reinigungsflüssigkeit nach den Ansprüchen 1 bis 4,
dadurch gekennzeichnet,
daß die Einsatzkonzentration des tensidischen Anteils 9 bis 50 Gew.-% beträgt.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 070 076 (THE PROCTER AND GAMBLE COMPANY) <br> * Seite 1 - Seite 12, Zeile 14 * <br> * Ansprüche 1-12; Beispiele 1,2,5,6,8,12 * <br> * Beispiele 14,16-19 * <br> --- | 1,2 | A61K7/50 <br> A61K7/08 |
| D,Y | EP-A-0 341 071 (UNILEVER PLC) <br> * das ganze Dokument * <br> --- | 1,2 | |
| D,A | EP-A-0 075 995 (THE PROCTER AND GAMBLE COMPANY) <br> * Seite 1 - Seite 9, Zeile 19 * <br> * Seite 13, Zeile 17 - Zeile 23 * <br> * Seite 15, Zeile 9 - Zeile 35 * <br> * Seite 16, Zeile 1 - Zeile 26 * <br> * Seite 17, Zeile 3 - Zeile 23 * <br> * Seite 21, Zeile 8 - Zeile 21 * <br> * Ansprüche 1,2; Beispiele 1-12 * <br> --- | 1-4 | |
| D,A | EP-A-0 370 312 (HENKEL KGAA) <br> * das ganze Dokument * <br><br> ----- | 1-4 | **RECHERCHIERTE SACHGEBIETE (Int. Cl.5)** <br><br> A61K |

Der vorliegende Recherchenbericht wurde für alle Patentansprüche erstellt

| Recherchenort | Abschlußdatum der Recherche | Prüfer |
|---|---|---|
| DEN HAAG | 06 MAERZ 1992 | SIERRA GONZALEZ |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................
& : Mitglied der gleichen Patentfamilie, übereinstimmendes
    Dokument

EPO FORM 1503 03.82 (P0403)